(19) Europäisches Patentamt
European Patent Office
Office européen des brevets

(11) Publication number: **0 434 844 A1**

(12) **EUROPEAN PATENT APPLICATION published in accordance with Art. 158(3) EPC**

(21) Application number: **90909386.6**

(22) Date of filing: **25.06.90**

(86) International application number: **PCT/JP90/00823**

(87) International publication number: **WO 91/00273 (10.01.91 91/02)**

(51) Int. Cl.[5]: **C07D 209/08**, C07D 209/12, C07D 209/42, C07D 223/16, C07D 225/06, C07D 267/14, C07D 267/22, C07D 281/10, C07D 281/18, A61K 31/395, A61K 31/40

(30) Priority: **26.06.89 JP 163145/89**

(43) Date of publication of application: **03.07.91 Bulletin 91/27**

(84) Designated Contracting States: **BE CH DE ES FR GB IT LI NL SE**

(71) Applicant: **KYORIN PHARMACEUTICAL CO., LTD.**
**No. 5, Kanda Surugadai 2-chome**
**Chiyoda-ku Tokyo(JP)**

(72) Inventor: **MIYASHITA, Mitsutomo**
**9994, Okaya-shi**
**Nagano 394(JP)**
Inventor: **KOHNO, Yasushi**
**1-10-5, Akatsuki**
**Oyama-shi, Tochigi-ken 329-02(JP)**
Inventor: **KOJIMA, Eisuke**
**2412-3, Ooaza-Nakata**
**Koga-shi, Ibaragi-ken 306(JP)**
Inventor: **SAITO, Koji**
**1-6-8, Akatsuki**
**Oyama-shi, Tochigi-ken 329-02(JP)**

(74) Representative: **TER MEER - MÜLLER - STEINMEISTER & PARTNER**
**Mauerkircherstrasse 45**
**W-8000 München 80(DE)**

(54) **NEW CYCLIC ANTHRANILIC ACID DERIVATIVE AND METHOD OF PRODUCING THE SAME.**

(57) Cyclic anthranilic acid derivatives of general formula (I) and acid addition salts and alkali salts thereof, useful for treating rheumatism, autoimmune diseases and metabolic bone diseases, wherein $R^1$, $R^2$ and $R^3$ may be the same of different from one another and each represents hydrogen, halogen, lower ($C_1$ to $C_3$) alkyl, lower ($C_1$ to $C_3$) alkoxy, amino, nitro, hydroxy, sulfonamide, trifluoromethyl, cyano, carboxyl, carbamoyl, acetyl, optionally substituted benzoylmethyl, methylthio, optionally substituted phenylethynyl, optionally substituted ethynyl, lower ($C_1$ to $C_3$) alkanoylamino, optionally substituted benzoylamino, lower ($C_1$ to $C_3$) alkylsulfonylamino, or optionally substituted phenylsulfonylamino; $R^4$ and $R^5$ may be the same or different from each other and each represents hydrogen, lower ($C_1$ to $C_3$) alkyl, cyano, carboxyl, hydroxylmethyl, optionally substituted phenyl, or benzyl; $R^6$ represents hydrogen, lower ($C_1$ to $C_3$) alkyl or benzyl; X represents a single bond, $-CHR^7-CHR^8-(CHR^9)_m-$, $-O-CHR^8-(CHR^9)_m-$, $-S(O)_n-CHR^8-(CHR^9)_m-$ or $-CO(CHR^8)_p-(CHR^8)_m-$; $R^7$, $R^8$ and $R^9$ may be the same or different from one another and each represents hydrogen, lower ($C_1$ to $C_3$) alkyl, cyano, carboxyl, hydroxymethyl, or optionally substituted phenyl; m and p are each 0 or 1; and n is an integer of 0 to 2; provided that $R^2$ represents neither chlorine nor hydrogen when both of $R^7$ and $R^8$ are hydrogen atoms and m is 0.

EP 0 434 844 A1

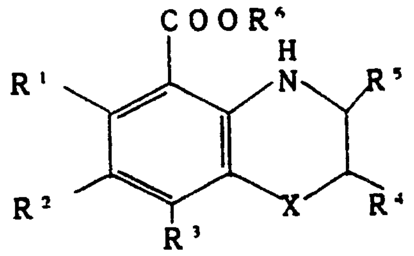

[1]

# NOVEL CYC1IC ANTHRANILIC ACID DERIVATIVES AND PROCESS OF PREPARING THE SAME

## Fie1d of the Technique

The present invention concerns nove1 cyc1ic anthrani1ic acid derivatives, their acid addition sa1ts or a1ka1i salts which have immuno-modulating action and strong inducing ability of suppressor T-ce11 as well as curing effect to rheumatic arthritis, preparing process thereof, and antirheumatic drug and immunodisease curing drug containing them as an active ingredient.

## Background of the Technique

Chronic diseases re1ated to immune responses include rheumatoid arthritis and other autoimmune diseases (systemic 1upus erythematosus, psoriatic arthritis, atopic dermatitis, ankylosing spondy1itis). These diseases are considered to be caused by bacteria, virus or autoantigens or by an aberration in cytokine regu1ation of T cells. Especially, patients with rheumatoid arthritis demonstrate vairous immune abnorma1ities including reduced functions of suppressor T cells and hyperactivity of B cells.

Non-steroidal antiinf1ammatory drugs are wide1y used as first choice drugs in the therapy of rheumatoid arthritis and other diseases due to immuno1ogical disorders. While these drugs offer symptomatic re1ief for patients with these diseases, they fail to alter the under1ying immuno1ogica1 dysfunction or the overa11 course of the disease process. Furthermore, serious side effects from prolonged use of these drugs also have been we11 documented.

On the other hand, second choice antirheumatic drugs, such as gold sa1t and D-penici11amine have 1itt1e acute antiinf1ammatory effects, but they appear to slow or halt the tissue destruction and more especia11y the progression of articu1ar damage. They also have immunomodu1atory effects. However, it is necessary to improve the safety and other aspects of these drugs, because of the higher incidence of side effects that have been observed in 40-50 % of the patients treated with these drugs.

Metabolic bone diseases as generic term inc1ude osteoporosis, osteomalacia and ostetic fibrous. In patients with the diseases, there are morbid changes in weight, constitution and structure of bone as a resu1t of the failure of the systemic bone formation and resorption process. This is caused by abnorma1ities in the somato1ogica1 regulatory system due to various hormones or vitamins and by congenita1 or acquired abnorma1ities of the functions of the osteocytes. It is a1so associated with abnormal ca1cium and phosphorus metabo1ism. Vitamin D, calcium, calcitonin and phosphorus are used as therapeutic drugs, but their effectiveness has not been c1ear1y proven and deve1opment of a superior drug has been strongly desired.

## Discl osure of Invention

As the resu1t of earnest investigations on the deve1opment of antirheumatism agent the inventors of the present invention have found that the novel cyclic anthrani1ic acid derivatives their acid addition sa1ts or a1ka1i sa1ts represented by a genera1 formu1a [I] of a category different from the known drugs have immuno-modu1ating action and strong inducing ability of supressor T-cell, and further have exce11ent curing effect to rheumatic arthritis and high safety condition.

Furthermore, the inventors of the present invention have found that the compounds of the present invention have inhibitory action against destruction of bone, which has 1ed to the completion of the present invention.

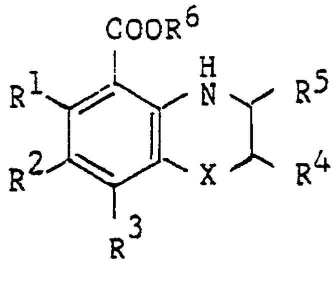

[I]

wherein $R^1$, $R^2$ or $R^3$, being same or different each other, denotes hydrogen atom,ha1ogen atom, 1ower

alkyl group of carbon number 1 - 3, lower alkoxy group of carbon number 1 - 3, amino group, nitro group, hydroxyl group, sulfonamide group, trifluoromethyl group, cyano group, carboxyl group, carbamoyl group, acetyl group, benzoylmethyl group which may be substituted, methylthio group, phenylethynyl group which can be substituted, ethynyl group which may be substituted, lower alkanoylamino group of carbon number l - 3, benzoylamino group which may be substituted, lower alkylsulfonylamino group of carbon number 1 - 3 or phenylsulfonylamino group which can be substituted respectively, $R^4$ or $R^5$, being same or different, denotes hydrogen atom, lower alkyl group of carbon number 1 - 3, cyano group, carboxyl group, hydroxymethyl group, phenyl group which can be substituted or benzyl group which can be substituted, respectively, $R^6$ denotes hydrogen atom, lower alkyl group of carbon number l - 3 or benzyl group x denotes single bond or denotes
- $CHR^7$ - $CHR^8$ - $(CHR^9)$ m -, -O-$CHR^8$ - $(CHR^9)$ m -, -S (O) n -$CHR^8$ - $(CHR^9)$ m - or -CO $(CHR^8)$ p - $(CHR^9)$ m - respectively, herein $R^7$, $R^8$ or $R^9$, being same or different each other, denotes hydrogen atom, lower alkyl group of carbon number l - 3, cyano group, carboxyl group, hydroxymethyl group, phenyl group which can be substituted, respectively, m or p denotes 0 or l respectively, and n denotes an integer of 0 - 2, provided $R^2$ can not be chlorine atom or hydrogen atom in case both $R^7$ and $R^8$ are hydrogen atom and m is equal to 0.

Besides, as the lower alkyl of lower alkyl group, lower alkoxy group, lower alkanoyl group, lower alkylsulfonylamino group etc., those of carbon number l - 3 of straight chain or branched chain such as for example methyl, ethyl, n-propyl, iso-propyl etc. can be exemplified. Further as the halogen atom, for example fluorine, chlorine, bromine or iodine can be exemplified. As "the phenyl which can be substituted" in benzoylmethyl group which can be substituted, phenylsulfonylamino group which can be substituted, benzoylamino group which can be substituted, phenylethynyl group which can be substituted and so on, those having one to three halogen atoms, lower alkyl group, lower alkoxy group or hydroxyl group at optional position on the benzen ring thereof can be exemplified.

Next, the processes for preparing the compound of the present invention are explained as follows. The so-called cyclic anthranilic acid has been synthesized by a process of oxidizing 1,7-trimethyleneisatin compounds with hydrogen peroxide in alkali (E. Ziegler et al., Monatsh. Chem., 94, 698 (1963), ibid 95, 59 (1964), USP 3794646) and by other processes such as reduction or cyclization reaction of quinoline-8-carboxylic acid (C. Satyendranath et al., J. Annamalai Univ., 2, 227 (1933), L.S. Povarov et al., Izv. Akad. Naak SSSR, Serkhim, 144 (l966), C. G. Wad et al., J, Heterocycl. Chem., 2, 4l4 (1965) ). However, in any case of them, only extremely restricted compounds are known and no report on pharmacological activities thereof has been recognized.

According to the present invention, a compound represented by the general formula [I] can be prepared through the following route.

Firstly, a compound of the general formula [I] wherein $R^6$ is hydrogen, i.e. a compound represented by a general formula [III] can be prepared by oxidizing a compound represented by a general formula [II] after alkali-treatment thereof. Typically speaking, it can be prepared by hydrolizing the compound represented by the general formula [II] with adding somewhat excess amount of a suitable base, for example, aqueous solution of sodium hydroxide or potassium hydroxide in a suitable solvent, for example, water or alcohol containing water, followed by oxidation thereof with not less than equimolecular amount of a mild oxidant, for example, aqueous solution of hydrogen peroxide or peracetic acid etc. It is preferable that the reaction temperature is 0 - 50 °C and the reaction period is 30 minutes to 3 hours.

In case X represents -S-$CHR^8$-$(CHR^9)$m- in the general formula [II] , a compound wherein S remains S, that wherein S changes to sulfinyl group and that wherein S changes to sulfonyl group, respectively, can be predominantly produced by controlling added amount of the oxidant and the working temperature. A compound of the general formula [II] which is a starting compound for the present process of preparation can be easily produced by a process similar to that of Japanese Kokai Sho 60-243088.

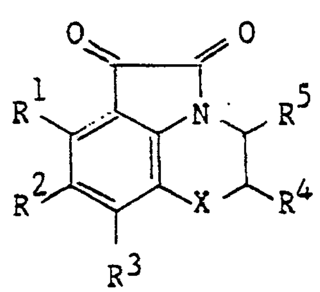

[II]

wherein $R^1$, $R^2$, $R^3$, $R^4$, $R^5$ and X are as above-mentioned.

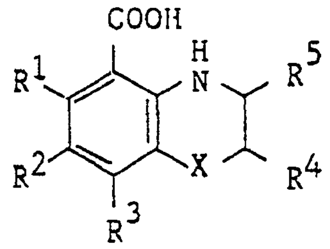

[III]

wherein $R^1$, $R^2$, $R^3$, $R^4$, $R^5$ and X are as above-mentioned.

Next, a compound of the genera1 formula [I] wherein $R^6$ is lower alkyl group of carbon number I - 3 or benzyl group, i.e. a compound of a genera1 formula [IV] can be produced by allowing a compound represented by the above-mentioned genera1 formula [III] to react with ha1ogenated lower alkyl or benzyl ha1ide. Typica11y speaking, it can be produced by a11owing a suitable alkyl halide, for examp1e, methy1 iodide, ethyl iodide etc. or benzyl halide to act on a compound of the genera1 formu1a [III] in a suitable solvent, for examp1e, dimethylformamide, dimethylsulfoxide, acetone, dioxane etc. and in the presence of a suitable base, for examp1e, sodium carbonate, potassium carbonate, potassium bicarbonate etc. at room temperature or on heating with stirring.

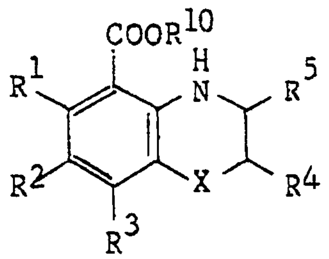

[IV]

wherein $R^1$, $R^2$, $R^3$, $R^4$, $R^5$ and X are as above-mentioned, $R^{10}$ denotes 1ower a1kyl group or benzyl group.

Further, a compound of the genera1 formula [III] can be produced by subjecting a compound of the general formula [IV] to hydrolysis or cata1ytic reduction. Typica11y speaking, in case $R^{10}$ is lower alky1 group of carbon number 1 - 3, it can be produced by hydrolizing a compound of the general formu1a [IV] in a suitable solvent, for example, methanol or ethanol etc. and in the presence of a suitab1e base, for example, sodium hydroxide or potassium hydroxide etc. at room temperature or on heating with stirring. In case $R^{10}$ is benzy1 group, it can be produced by subjecting a compound of the genera1 formula [IV] to cata1ytic reduction in a suitable solvent, for example, dimethy1formamide etc. and in the presence of a suitable cata1yst, for examp1e, 10 % pa11adium carbon.

Furthermore, a compound of the general formula [I] wherein X is $-S-CHR^8-(CHR^9)m-$, i.e. a compound of the general formu1a [IV] can be produced by subjecting a compound of the general formula [I] wherein X is $-SO-CHR^8-(CHR^9)m-$ or $-SO_2CHR^8-(CHR^9)m-$, i.e. a compound of a general formu1a [V] to reduction. Typica11y speaking, it can be produced by subjecting a compound of the general formula [V] to reduction in a suitable solvent, for examp1e, methanol, water or methylene ch1oride etc. with employing a suitable reducing agent, for examp1e, stannous chloride, titanium trich1oride or diphosphor tetraiodide etc., if circumstances require, sodium borohydride or 1ithium aluminum hydride etc. at room temperature or on heating with stirring.

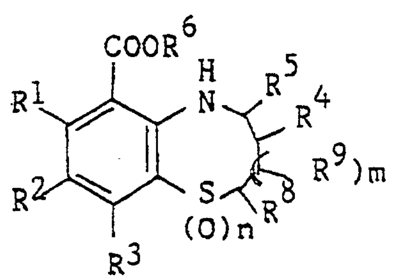

[V]

wherein $R^1$, $R^2$, $R^3$, $R^4$, $R^5$, $R^6$, $R^8$, $R^9$ and m are as above-mentioned, n denotes one or two.

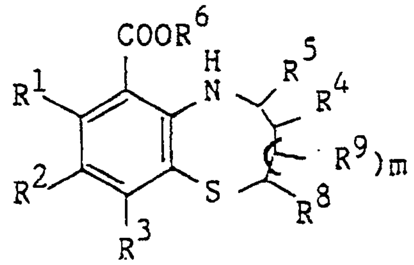

[VI]

wherein $R^1$, $R^2$, $R^3$, $R^4$, $R^5$, $R^6$, $R^8$, $R^9$ and m are as above-mentioned.

Furthermore, a compound of the general formula [V] can be produced by oxidizing a compound of the general formula [VI]. Typically speaking, it can be produced by oxidizing a compound of the general formula [VI] in the presence of equal amount or excess amount of a mild oxidant, for example, m-chloroperbenzoic acid or aqueous solution of hydrogen peroxide etc. and in a suitable solvent, for example, methylene chloride, or alcohol etc. at room temperature or on heating with stirring.

Moreover, a compound of the general formula [I] wherein X is $-SO_2CHR^8-(CHR^9)m$, i.e. a compound of a general formula [VIII] can be prepared by oxidizing a compound of the general formula [I] wherein X is $SOCHR^8-(CHR^9)m$, i.e. a compound of a general formula [VII]. Typically speaking, it can be produced by oxidizing a compound of the general formula [VII] in the presence of equal amount or excess amount of a mild oxidant, for example, m-chloroperbenzoic acid or aqueous solution of hydrogen peroxide and in a suitable solvent, for example, methylene chloride or alcohol etc. at room temperature or on heating with stirring.

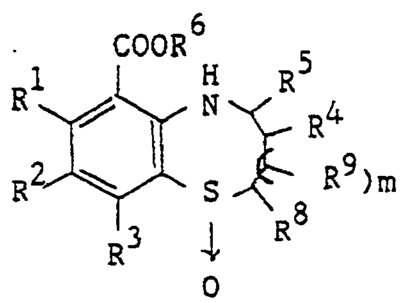

[VII]

wherein $R^1$, $R^2$, $R^3$, $R^4$, $R^5$, $R^6$, $R^8$, $R^9$ and m are as above-mentioned.

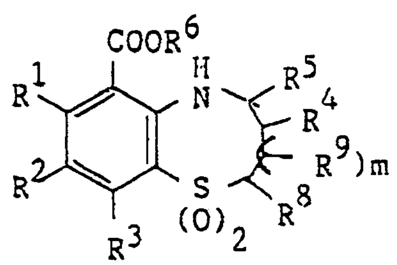

[VIII]

wherein $R^1$, $R^2$, $R^3$, $R^4$, $R^5$, $R^6$, $R^8$, $R^9$ and m are as above-mentioned.

Moreover, a compound of the general formula [I] wherein $R^2$ is amino group, i.e. a compound of a general formula [X] can be produced by subjecting a compound of the general formula [I] wherein $R^2$ is nitro group, i.e. a compound of a general formula [IX] to catalytic reduction. Typically speaking, it can be produced by subjecting a compound of the general formula [IX] to catalytic reduction in a suitable solvent, for example, dimethylformamide etc. and in the presence of a suitable catalyst, for example, 10 % palladium carbon.

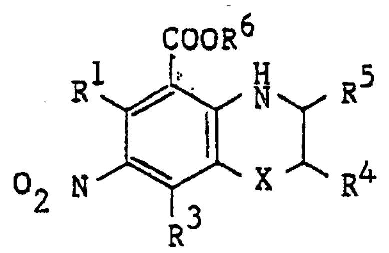

[IX]

wherein $R^1$, $R^3$, $R^4$, $R^5$, $R^6$ and X are as above-mentioned.

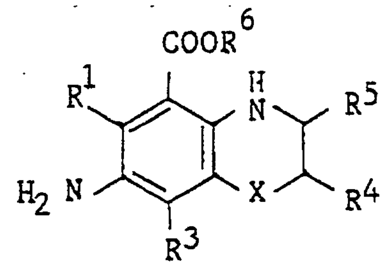

[X]

wherein $R^1$, $R^3$, $R^4$, $R^5$, $R^6$ and X are as above-mentioned.

Moreover, a compound of the genera1 formula [I] wherein $R^2$ is lower alkanoyla1ky1 group of carbon number 1 - 3, benzoylamino group which can be substituted, 1ower alky1sufonylamino group of carbon number I - 3 or phenylsufony1amino group which can be substituted, i.e. a compound of a general formula [XII] can be prepared by acting a compound of the genera1 formula [XI] on a compound of the general formula [I] wherein $R^2$ is amino group, i.e. a compound of the genera1 formu1a [X]. Typica11y speaking, it can be produced by acting ha1ogenide of the genera1 formu1a [XI] on a compound of the general formula [X] in a suitable solvent, for example, dioxane, dimethylsulfoxide etc. and in the presence of a suitab1e base for example triethylamine pyridine etc with stirring at room temperature.

$R^{11}$- Y [XI]

wherein $R^{11}$ denotes 1ower a1kanoy1 group of carbon number I - 3, benzoy1 group which can be substituted, lower a1ky1su1fony1 group of carbon number 1 - 3 or phenylsulfony1 group which can be substituted, Y denotes ha1ogen atom.

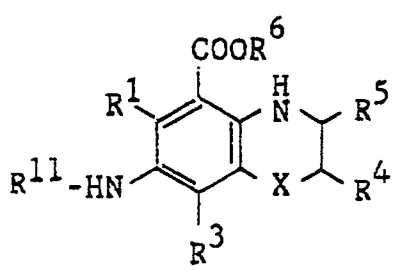

[XII]

wherein $R^1$, $R^3$, $R^4$, $R^5$, $R^6$, $R^{11}$ and X are as above-mentioned.

Moreover, a compound of the genera1 formula wherein $R^2$ is cyano group, i.e. a compound of a general formu1a [XIV] can be produced by acting cyanating agent on a compound of the genera1 formula [I] wherein $R^2$ is bromine atom, i.e. a compound of a genera1 formula [XIII]. Typically speaking, it can be produced by acting a suitable cyanating agent, for example, copper cyanide, potassium cyanide, sodium cyanide etc. on a compound of a genera1 formula [XIII] in a suitab1e so1vent, for examp1e, dimethy1formamide, pyridine, N-methy1pyrro1idone on heating with stirring.

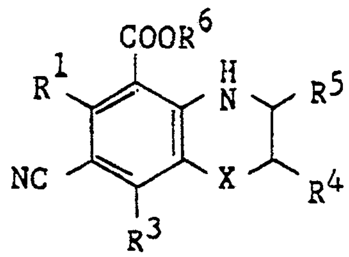

[XIV]

wherein $R^1$, $R^3$, $R^4$, $R^5$, $R^6$ and X are as above-mentioned.

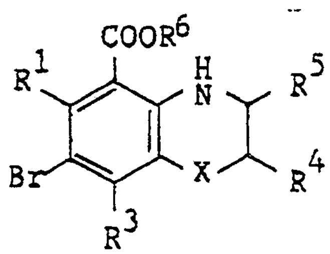

[XIII]

wherein $R^1$, $R^3$, $R^4$, $R^5$, $R^6$ and X are as above-mentioned.

Moreover, a compound of the general formula [I] wherein $R^2$ is carbomoyl group or carboxy1 group, i.e. a compound of a genera1 formu1a [XV] can be prepared by hydrolizing a compound of the genera1 formu1a [I] wherein $R^2$ is cyano group, i.e. a compound of a genera1 formu1a [XIV]. Typica11y speaking, it can be produced by hydrolizing a compound of the genera1 formu1a [XIV] in a suitab1e solvent, for examp1e, ethano1, methano1 etc. with a suitab1e base, for example, sodium hydroxide, potassium hydroxide etc.

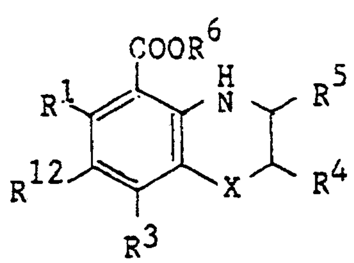

[XV]

wherein $R^1$, $R^3$, $R^4$, $R^5$, $R^5$ and X are as above-mentioned, $R^{12}$ denotes carbomoyl group or carboxyl group.

Moreover, a compound of the genera1 formula [I] wherein $R^2$ is pheny1ethyny1 group which can be substituted or ethynyl group which can be substituted, i.e. a compound of a genera1 formu1a [XVII] can be produced by acting phenylacetylene which can be substituted or acety1ene which can be substituted on a compound of a genera1 formula [XVI]. Typica11y speaking, it can be produced by heating a compound of a general formu1a [XVI] and phenylacety1ene which can be substituted or acety1ene which can be substituted with triethy1amine and copper iodide in a suitable so1vent, for example, dimethy1formamide, tetrahydrofuran etc. and in the presence of bis-tripheny1phosphine palladium diacetate, bistripheny1phosphine palladium chloride etc.

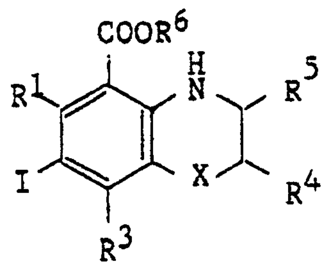

[XVI]

wherein $R^1$, $R^3$, $R^4$, $R^5$, $R^6$ and X are as above-mentioned.

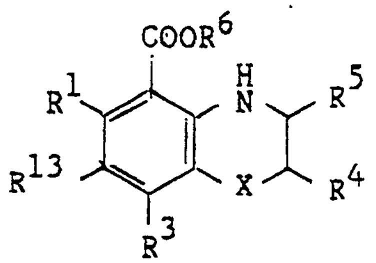

[XVII]

wherein $R^1$, $R^3$, $R^4$, $R^5$, $R^6$ and X are as above-mentioned, $R^{13}$ denotes phenylethynyl group which can be substituted, ethynyl group which can be substituted.

Moreover, a compound of the general formula [I] wherein $R^2$ is acetyl group or benzoylmethyl group which can be substituted, i.e. a compound of a general formula [XVIII] can be produced by subjecting a compound of the general formula [I] wherein $R^2$ is phenylethynyl group which can be substituted or ethynyl group which can be substituted, i.e. a compound of the general formula [XVII] to hydration. Typically speaking, it can be produced by heating a compound of the general formula [XVII] with concentrated sulfuric acid, mercuric sulfate etc. in a suitable solvent, for example, acetone containing water.

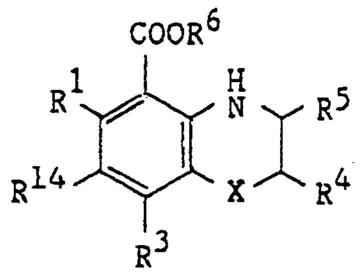

[XVIII]

wherein $R^1$, $R^3$, $R^4$, $R^5$, $R^6$ and X are as above-mentioned, $R^{14}$ denotes benzoylmethyl group, acetyl group.

Next, a compound represented by the general formula [I], if desired, can be converted into a salt thereof according to the conventional process. As an acid addition salt thereof, a salt of inorganic acid, for example, hydrochloric acid, sulfuric acid, phosphoric acid etc. or a salt of organic acid such as methansulfonic acid, lactic acid, acetic acid, citric acid, tartaric acid etc. can be exemplified. Then, as an alkali salt thereof, a metallic salt thereof such as sodium salt, potassium salt etc. can be exemplified.

The Best Mode to execute the Present Invention

The concrete examples of the present invention are explained as follows, but the present invention suffers no restriction by these examples.

Example I

N-acetyl-7-amino-5-bromoindoline

To a mixed solution of ethanol (400 ml) and water (100 ml), were added N-acetyl-5-bromo-7-nitroindoline

(I33.1 g), iron powder (I0 mesh; 75 g) and concentrated hydrochloric acid (5 m1), and the mixture was subjected to refluxing for 5 hours with vigorous stirring. The reaction mixture was filtered by suction while hot to remove insoluble matter, and then the filtrate thereof was concentrated and cooled on standing to give the intended compound 38.3 g (32.2 %) as a brown needle crystal. M.P.: 154 - 155 °C

Example 2

N-acetyl-5-bromo-7-cyanoindoline

Into 6 N hydrochloric acid (I78.5 m1) was suspended N-acetyl-7-amino-5-bromoindoline (51.0 g), and aqueous solution (212 m1) of sodium nitrite (2I.2 g) was dropwise added thereto under cooling with stirring over 30 minutes and at the inside temperature of not higher than -I °C. After stirred for 30 minutes at the temperature as such, sodium carbonate was added to the reaction mixture to neutralize it. This reaction mixture was poured into aqueous solution (200 ml) of copper cyanide (27.5 g) and sodium cyanide (30.7 g), and the mixture was allowed to stand for one night at room temperature. The reaction mixture was again subjected to stirring for 30 minutes at the inside temperature of 50 °C and was cooled to give a deposited crystal, which was then collected by filtration, washed with water and dried. The crude crystal was recrystallized from acetonitrile to give the intended compound 43.0 g (8I.I %). M.P.: 170 - I75 °C

Example 3

5-Bromoindoline-7-carboxylic acid

After adding N-acety1-5-bromo-7-cyano-indoline (17.7 g) to concentrated hydrochloric acid (I77 ml), the mixture was subjected to refluxing for one hour with stirring. The reaction mixture was cooled with ice to give deposited crystal, which was then collected by filtration. The combined crystal and crude crystal obtained by concentrating the mother liquor were suspended into water (50 m1) to which thereafter 50 % aqueous solution of sodium hydroxide was added, and then the insoluble matter was collected by filtration. To the filtrate was added concentrated hydrochloric acid, and the deposited crystal was collected by filtration. Further, after repeating this operation again, the obtained crystal was dried to give the intended compound II.2 g (69.6 %) as light yellow powder. M.P.: 212.5 - 123.5 °C (decomp.)

| Elementary analysis (%) as $C_9H_8BrNO_2$ | | | |
|---|---|---|---|
| Calculated value | C: 44.66 | H: 3.33 | N: 5.79 |
| Observed value | C: 44.71 | H: 3.24 | N: 5.78 |

Example 4

N-Acety1-7-cyano-5-nitroindoline

To a mixed solution of acetic acid (3.8 m1), concentrated sulfuric acid (4.7 m1) and funing nitric acid (0.8 m1), under cooling with stirring, were added only small portions of N-acety1-7-cyano-indoline (2.42 g). After stirred at the temperature as such for 7.5 hours, the reaction mixture was poured into ice-water (50 ml). The deposited crystal was collected by filtration, washed with water and acetone, and then dried to give the intended compound 2.6 g (66.7 %) as white powder. M.P.: 245 - 255 °C.

Example 5

5-Nitroindoline-7-carboxylic acid

Into concentrated hydrochloric acid (63.6 ml) was dissolved N-acetyl-7-cyano-5-nitroindoline (5.3 g), which was then subjected to stirring at 140 °C for 45 minutes. The reaction mixture was cooled to 0 °C to give deposited crystal, which was then collected by filtration The obtained crude crystal was suspended into water (50 m1), and was rendered basic by adding 50 % sodium hydroxide, and the insoluble matter was collected by filtration. After adding concentrated hydrochloric acid to the filtrate, the deposited crystal was collected by filtration and dried to give the intended compound 3.7 g (77.1 %) as yellow powder. M.P.: 294 - 297 °C (decomp.)

Elementary analysis (%) as $C_9 H_8 N_2 O_4$

| | | | |
|---|---|---|---|
| Calculated value | C: 51.93 | H: 3.87 | N: 13.46 |
| Observed value | C: 51.80 | H: 3.83 | N: 13.55 |

Example 6

4,5,6,7-Tetrahydroazepino [3,2,1-hi] indole-l,2-dione
To a solution of oxa1ic chloride (27.5 m1) in tetrahydrofuran (THF; 500 ml) which was subjected to refluxing with heating, was dropwise added a solution of 2,3,4,5 - tetrahydro - 1H - 1 - benzazepine (28.8 g) in THF (l00 m1), which was thereafter subjected to stirring for 4 hours as such. After cooled, the solvent was disti11ed off and the residue was dissolved into carbon disulfide (600 m1), to which then on1y sma11 portions of aluminium chloride (52.27 g) were added at room temperature with stirring and was further subjected to stirring for 4 hours. With remova1 of supernatant solution from the reaction mixture, ice-water was added to the residue, which was then extracted with chloroform. The organic layer was thoroughly washed with water, dried over anhydrous sodium su1fate and then the solvent thereof was distilled off.

The residue was recrystallized from acetonitril to give the intended compound 28.32 g (7l.9 %) as dark red crystal. M.P.: l32 °C

Elementary analysis (%) as $C_{12} H_{11} NO_2$

| | | | |
|---|---|---|---|
| Calculated value | C: 71.63 | H: 5.51 | N: 6.96 |
| Observed value | C: 72.10 | H: 5.63 | N: 7.05 |

Example 7

9-Bromo-4,5,6,7-Tetrahydroazepino [3,2,1-hi] indo1e-1,2-dione
4,5,6,7-Tetrahydroazepino [3,2,1-hi] indo1e-1,2-dione (10 g) and N-bromosuccinic imide (11.17 g) were dissolved into dimethy1formamide (DMF; 100 ml) and, after stirred for one flour at 80 °C, the solvent was disti11ed off. The residue was dissolved into chloroform, washed with water, dried over anhydrous sodium sulfate and thereafter the solvent was distilled off to give the intended compound l3.99 g (95.5 %), which was then recrysta11ized from acetonitril to give dark red needle crysta1. M.P.: l59 - 160 °C

Elementary analysis (%) as $C_{12} H_{10} Br NO_2$

| | | | |
|---|---|---|---|
| Calculated value | C: 51.45 | H: 3.60 | N: 5.00 |
| Observed value | C: 51.33 | H: 3.56 | N: 5.10 |

Example 8

9-Nitro-4,5,6,7-Tetrahydroazepino [3,2,l-hi] indole-1,2-dione
After 2,3,4,5-tetrahydro-lH-l-benzazepine (3.42 g) was added under coo1ing with ice to fuming nitric acid (8 m1) and the mixture was subjected to stirring for one hour at the same temperature, the temperature thereof was put back to room temperature and the mixture was stirred for one hour. The reaction mixture was poured into ice-water (100 ml), and the deposited crysta1 was collected by filtration, washed with water

and dried to give the intended compound 3.97 g (90%), which was then recrystallized from acetonitril to give orange color crystal. M.P.: l53-l54 °C

Elementary analysis (%) as $C_{12}H_{10}N_2O_4$

| | | | |
|---|---|---|---|
| Calculated value | C: 58.54 | H: 4.09 | N: 11.38 |
| Observed value | C: 58.56 | H: 4.06 | N: 11.37 |

Example 9

7-Bromo-2,3,4,5-tetrahydro-1H-1-benzazepine-9-carboxylic acid
9-Bromo-4,5,6,7-tetrahydroazepino [3,2,l-hi] indole-l,2-dione (13.99 g) and sodium hydroxide (10 g) were dissolved into water (800 m1), to which then 35 % aqueous so1ution of hydrogen peroxide (10 ml) was added, and the mixture was stirred for 4 hours at room temperature. The mixture was rendered to have pH 2 with concentrated hydrochloric acid, and the deposited crystal was collected by fi1tration, washed with water and dried to give the intended compound 10.24 (76.4 %.), which was then recrystallized from ethyl acetate-n-hexane to give yellow needle crystal. M.P.: 157 - 158 °C

Elementary analysis (%) as $C_{11}H_{12}BrNO_2$

| | | | |
|---|---|---|---|
| Calculated value | C: 48.91 | H: 4.48 | N: 5.19 |
| Observed value | C: 48.62 | H: 4.40 | N: 5.12 |

Example 10

7-Nitro-2,3,4,5-tetrahydro-1H-1-benzazepine-9-carboxy1ic acid

Likewise as in Examp1e 9, the intended compound was obtained in yield of 80.6 % from 9-nitro-4,5,6,7-tetrahydroazepino (3,2, 1-hi] indole-l,2-dione. M.P.: 224-225 °C

Elementary analysis (%) as $C_{11}H_{12}N_2O_4$

| | | | |
|---|---|---|---|
| Calculated value | C: 55.93 | H: 5.12 | N: 11.86 |
| Observed value | C: 55.88 | H: 5.15 | N: 11.83 |

Example 11

7-Cyano-2,3,4,5-tetrahydro-1H-1-benzazepine-9-carboxylic acid

a) 7-Bromo-2,3,4,5-tetrahydro-lH-1-benzazepine-9-carboxy1ic acid benzyl ester
To 7-bromo-2,3,4,5-tetrahydro-1H-1-benzazepine-9-carboxylic acid (8.53 g) dissolved in DMF (80 ml), were added potassium carbonate (13.8 g) and benzy1 bromide (4.5 ml) and the mixture was stirred for 2 hours at room temperature. After the inso1ub1e matter was filtered off and the filtrate was concentrated, a mixed 1iquid of ethyl acetate-benzene (1:1, 400 ml) was added to the residue, and the mixture was washed with water, dried over anhydrous sodium su1fate and then subjected to disti11ing off the solvent to give the intended compound 11.35 g (99.7 %) as brown oi1y matter.

b) 7-Cyano-2,3,4,5-tetrahydro-lH-l-benzazepine-9-carboxy1ic acid benzyl ester
The ester compound (ll.35 g) obtained in a) was dissolved in DMF (40 ml), added with cuprous cyanide (3.75 g), and the mixture was refluxed for 8 hours. After cooled, ferrous chloride•6 hydrate (l0 g), water (100 m1) and concentrated hydrochloric acid (2 ml) were added to the reaction mixture, which was then

stirred for 30 minutes at 60 °C and extracted with ethyl acetate. The organic 1ayer was washed successively with water, l0 % hydroch1oric acid, water, 5 % aqueous solution of sodium hydroxide and water, and dried over anhydrous sodium su1fate, which was thereafter subjected to distilling off the solvent. The residue was purified through silica-gel co1umn-chromatography (developing solvent; ethyl acetate: n-hexane = 1:3) to give the intended compound 6.81 g (70.6 %) as light yellow crystal.

c) 7-Cyano-2,3,4,5-tetrahydro-1H-1-benzazepine-9-carboxy1ic acid

The compound (3.30 g) obtained in b) was dissolved into ethanol (200 ml), to which then l0 % palladium-carbon (500 mg) was added, and was subjected to cata1ytic reduction at room temperature. After 4 hours, the cata1yst was filtered off and the fi1trate was concentrated to give the intended compound l.87 g (94 %), which was then recrystallized from ethyl acetate-n-hexane to give slightly yellow crystal. M.P.: l75 °C

Elementary analysis (%) as $C_{12}H_{12}N_2O_2$

| | | | |
|---|---|---|---|
| Calculated value | C: 66.65 | H: 5.59 | N: 12.95 |
| Observed value | C: 66.73 | H: 5.62 | N: 12.93 |

Example l2

2,3,4,5-Tetrahydro-1H-l-benzazepine-7,9-di-carboxy1ic acid

7-Cyano-2,3,4,5-tetrahydro-1H-1-benzazepine-9-carboxylic acid benzyl ester (3.30 g) and sodium hydroxide (2 g) in a mixed solution of water-ethanol (l:l, 60 ml) was refluxed for 8 hours. After cooled, water (l00 ml) was added to the reaction mixture, which was then rendered to have pH 3 with concentrated hydrochloric acid. The deposited crystal was collected by filtration, washed with water and dried to give the intended compound l.84 g (85.0 %), which was then recrysta1lized from ethanol to give slightly ye1low crysta1. M.P.: 287 - 288 °C

Elementary analysis (%) as $C_{12}H_{13}NO_4$

| | | | |
|---|---|---|---|
| Calculated value | C: 61.27 | H: 5.57 | N: 5.95 |
| Observed value | C: 61.29 | H: 5.58 | N: 6.43 |

Example 13

7-Methansu1fonylamino-2,3,4,5-tetrahydro-1H-l-benzazepine-9-carboxy1ic acid

a) 7-Nitro-2,3,4,5-tetrahydro-1H-1-benzazepine-9-carboxy1ic acid methyl ester

7-Nitro-2,3,4,5-tetrahydro-lH-l-benzazepine-9-carboxylic acid (6.25 g) was disso1ved into DMF (50 ml), to which then potassium carbonate (11 g) and methy1 iodide (4.9 ml) were added, and was stirred for 2 hours at room temperature. After the inso1ub1e matter was fi1tered off and the filtrate was concentrated, ethyl acetate (500 ml) was added to the residue, which was then washed with water and dried over anhydrous sodium sulfate. The solvent was distilled off to give the intended compound 6.65 g (quantitatively) as yellow crystal.

b) 7-Amino-2,3,4,5-tetrahydro- H-l-benzazepine-9-carboxylic acid methyl ester

The ester (6.65 g) obtained in a) was dissolved into DMF (200 ml), to which l0 % palladium-carbon (1 g) was then added, and was subjected to cata1ytic reduction at room temperature. After 7 hours, the cata1yst was filtered off and the filtrate was concentrated to give the intended compound 5.86 g (quantitatively) as brown oily matter.

c) 7-Methanesulfony1amino-2,3,4,5-tetrahydro-lH-l-benzazepine-9-carboxy1ic acid methyl ester

The ester (2.90 g) obtained in b), mesil chloride (MsC1, l.4 ml) and triethyl amino (2.5 m1) were disso1ved in doxane (80 m1), and the resultant solution was stirred for 6 hours at room temperature. Water (300 ml) was added to the reaction mixture, which was then extracted with ethyl acetate, dried over anhydrous sodium su1fate and subjected to disti1ling off the solvent to give the intended

compound 2.94 g (80.8 %) as brown oily matter.

d) 7-Methanesulfonylamino-2,3,4,5-tetrahydro-1H-1-benzazepine-9-carboxylic acid

The ester (2.94 g) obtained in c) and sodium hydroxide (2 g) were dissolved into a mixed solution of water-ethanol (1:1, 50 ml), and was refluxed for 4 hours. To the reaction mixture was added water (200 ml), which was then rendered to have pH 2 and extracted with ethyl acetate. The organic layer was washed with water, dried over anhydrous sodium sulfate and the solvent was distilled off to give the intended compound 1.48 g (50.3 %), which was then recrystallized from ethanol to give yellow crystal.

M.P.: 204 - 205 °C

Elementary analysis (%) as $C_{12}H_{16}N_2O_4S$

| | | | |
|---|---|---|---|
| Calculated value | C: 50.69 | H: 5.67 | N: 9.85 |
| Observed value | C: 50.43 | H; 5.65 | N: 9.60 |

Example I4

7-p-Toluenesulfonylamino-2,3,4,5-tetrahydro-IH-I-benzazepine-9-carboxylic acid

a) 7-p-Toluenesulfonylamino-2,3,4,5-tetrahydro-IH-I-benzazepine-9-carboxylic acid methyl ester

Employing 7-amino-2,3,4,5-tetrahydro-IH-I-benzazepine-9-carboxylic acid methyl ester with employment of p-tosyl chloride, which were allowed to react likewise as in Example 13 c), the intended compound was obtained in yield of 95.0 % as brown oily matter.

b) 7-p-Toluenesulfonylamino-2,3,4,5-tetrahydro-IH-I-benzazepine-9-carboxylic acid

The compound obtained in a) was allowed to react likewise as in Example I0 d) to give the intended compound in yield of 87.7 %, which was then recrystallized from ethanol to give slightly yellow crystal.

M.P.: 232 - 233 °C

Elementary analysis (%) as $C_{18}H_{20}N_2O_4S$

| | | | |
|---|---|---|---|
| Calculated value | C: 59.98 | H: 5.59 | N: 7.77 |
| Observed value | C: 60.09 | H: 5.62 | N: 7.85 |

With respect to the compound of the present invention, the results supporting the utility thereof are shown by the Experimental examples as follows.

Experimental example 1

Inhibition of increased vascular permeability

Male ddy mice (6 weeks of age) were used in this experiment. Example 9 compound suspended in 5 % gum arabic solution was administered p.o., 45 minutes before the i.v. injection of I.0 % Evans blue solution (0.1 ml/I0 g B.W.). Immediately after the i.v. injection, I.0 % acetic acid solution was administered i.p. (0.I ml/I0 g B.W.). The mice were killed 30 minutes after the acetic acid injection. Peritoneal exudate was collected by washing out the peritoneal cavity with physiological saline and centrifuged at 3,000 r.p.m. After the centrifugation, spectrophotometry of the supernatant was measured at 630 nm. Results were expressed as dye leakage and percent inhibition.

Table 1

| Compound | Dose (mg/kg) | N | Dye leakage (μg/ml) (mean ± S.E. | Inhibition (%) |
|---|---|---|---|---|
| Control | - | 8 | 25.77 ± 1.81 | - |
| Example 9 | 100 | 9 | 17.43 ± 1.38 * | 32.3 |

* Significantly different from control, $p<0.01$.

As shown in Table I, the compound of the present invention significantly inhibited the rise in vascular permeability caused by the injection of acetic acid.

Experimental Example 2

Inhibitory action against thermal denaturation of protein

Bovine serum albumin was dissolved into I/I5 M phosphoric acid buffer solution, which was then adjusted to 0.7 %. After adding the solution of the Present invention compound thereto, it was heated at 65 °C for 7 - 8 minutes. After cooled, the absorbance at 660 nm was measured, compared with the absorbance of the reference to derive the inhibitory rate and then the concentration necessary to inhibit 50 % was obtained.

Table 2

| | $IC_{50}$ (μM) |
|---|---|
| Example 9 | 132 |
| Example 10 | 71 |

As shown in Table 2, the inhibitory action against thermal denaturation of protein was obtained in the present invention compound.

Experimental example 3

Inhibitory action against bone resorption

According to Raisz et al method (J. Clin. Invest., 155, 103 (1965)), bone piece of front arm of mouse labelled with radioactive calcium ($^{45}$Ca) was prepared in order to measure bone resorption. That is, the radioactive calcium ($^{45}CaCl_2$; 200 μCi) was injected under the skin to the back of rats in the 17th day of pregnancy and two days later they were killed with exsanguination by cutting of the carotid artery thereof. After the embryos were picked up and washed with sterilized phosphated buffer solution, the bone of front arm was taken out, which was then rendered free of the attached muscle and cartilage of the both ends, and said piece of bone was cultivated in the culture medium of BGJb.
Next, according to the method of Tsuda et al (J. Bone Miner. Metab., I, 207 (1986)), the effect of the present invention compound against the bone resorption was determined, which are described as follows. The piece of bone was put on a milipore-filter, and was precultivated, in a well with 24 holes plate accomodating 0.6 ml culture medium of BGJb, at 37 °C and under 5 % carbon dioxide. After the precultivation for 24 hours, said bone with the filter were transferred into a test culture medium added with interleukin - 1β (1L-1β) or with both 1L-1β and drugs and the cultivation was continued. After 72 hours

cultivation in said test culture medium, 0.3 ml of the supernatant was taken out and $^{45}Ca$ released from the piece of bone was determined. After the piece of bone was immersed in 0.3 ml of 1 N hydrochloric acid for more than 1.5 hours, l0 ml of ACSII was added thereto and the radioactivity was determined.
Furthermore, the activity of bone resorption was represented by the ratio (percentage) of $^{45}Ca$ released in the supernatant of cultivation to the total radioactivity which had been contained in the piece of bone (the sum of radioactivities in the piece of bone and the supernatant of cultivation). One well with 24 holes plate was employed per one piece of bone and every experimental group was constituted by 5 - 6 pieces of bone of embryo obtained from one pregnant rat. The inhibitory rate against bone resorption was derived from the following formula.

$$\text{Inhibitory rate (\%)} = \frac{\text{(activity of IL-1}\beta\text{ addition group)} - \text{(activity of both IL-1}\beta\text{ and drugs addition group)}}{\text{(activity of IL-1}\beta\text{ addition group)} - \text{(activity of non-addition (reference) group)}} \times 100$$

The result was illustrated in Table 3.

Table 3

| | Concentration (μg/ml) | Inhibitory rate against bone resorption (%) |
|---|---|---|
| Example 11 c) | 25 | 54 |
| Example 12 | 25 | 84 |
| Ipriflavone * | 25 | 58 |

* ; 7-isopropoxy isoflavone

As obvious from the above, the present invention compound has a strong inhibotory action against bone resorption.

Utilizability on industrial field

As stated above, the cyclic anthranilic acid derivatives of the present invention have the immuno-modulating action and the strong inducing ability of suppresor T cell as well as curing effect to rheumatic arthritis, and are further suitable for a curing medicament of metabolic bone diseases.

## Claims

1. Cyclinc anthranilic acid derivatives, their acid addition salts or alkali salts thereof represented by a general formula [I]

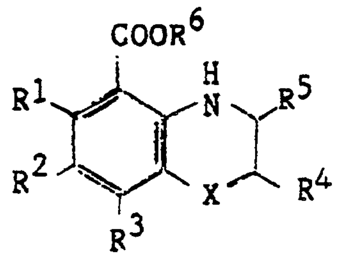

[I]

wherein $R^1$, $R^2$ and $R^3$, being same or different each other, denote hydrogen atom, halogen atom, lower alkyl group of carbon number 1 - 3, lower alkoxy group of carbon number 1 - 3, amino group, nitro group, hydroxyl group, sulfonamide group, trifluoromethyl group, cyano group, carboxyl group,

carbamoyl group, acety1 group, benzoyl-methy1 group which can be substituted, methylthio group, phenylethyny1 group which can be substituted, ethyny1 group which can be substituted, 1ower alkanoyl-amino group of carbon number 1 - 3, benzoyl-amino group which can be substituted, lower alkylsu1fonylamino group of carbon number I - 3 or pheny1sulfonylamino group which can be substituted, $R^4$ or $R^5$, being same or different each other, denotes hydrogen atom, lower alkyl group of carbon number 1 - 3, cyano group, carboxy1 group, hydroxymethy1 group, phenyl group which can be substituted or benzy1 group which can be substituted, $R^6$ denotes hydrogen atom, lower alky1 group of carbon number 1 - 3 or benzyl group, x denotes sing1e bond or denotes - $CHR^7$ - $CHR^8$ - $(CHR^9)m$ -, -O-$CHR^8$ - $(CHR^9)m$ -, - S(O)n(O)n - $CHR^8$ - $(CHR^9)m$ or - CO $(CHR^8)p$ - $(CHR^9)m$ -, herein $R^7$, $R^8$ or $R^9$, being same or different each other, denotes hydrogen atom, lower alkyl group of carbon number 1 - 3, cyano group, carboxyl group, hydroxymethyl group or phenyl group which can be substituted, m or p denotes 0 or 1 respectively, and n denotes an integer of 0 - 2, provided $R^2$ can not be chlorine atom or hydrogen atom in case both $R^7$ and $R^8$ are hydrogen atom and m is equa1 to 0.

2. A process of preparing cyc1ic anthranilic acid derivatives, their acid addition sa1ts or alka1i salts thereof represented by a genera1 formula [III]

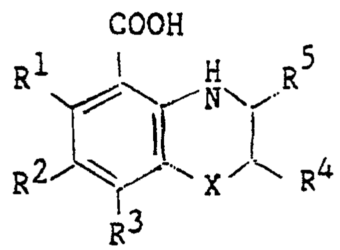

[III]

wherein $R^1$, $R^2$ and $R^3$, being same or different each other, hydrogen atom, halogen atom, 1ower a1kyl group of carbon number I - 3, lower alkoxy group of carbon number I - 3, amino group, nitro group, hydroxyl group, su1fonamide group, trifluoromethyl group, cyano group, carboxyl group, carbamoy1 group, acetyl group, benzoy1methy1 group which can be substituted, methy1thio group, pheny1ethyny1 group which can be substituted, ethyny1 group which can be substituted, lower alkanoylamino group of carbon number 1 - 3, benzoylamino group which can be substituted, 1ower a1kylsu1fony1amino group of carbon number I - 3 or phenylsulfonylamino group which can be substituted, $R^4$ or $R^5$, being same or different each other, denotes hydrogen atom, 1ower a1kyl group of carbon number I - 3, cyano group, carboxyl group, hydroxymethyl group, phenyl group which can be substituted or benzyl group which can be substituted, and x denotes single bond or denotes - $CHR^7$ - $CHR^8$ - $(CHR^9)m$ -, O - $CHR^8$ - $(CHR^9)m$ -, - S(O)n - $CHR^8$ - $(CHR^9)m$ or - CO $(CHR^8)p$ - $(CHR^9)m$ -, herein $R^7$, $R^8$ or $R^9$, being same or different each other, denotes hydrogen atom, 1ower a1ky1 group of carbon number 1 - 3, cyano group, carboxy1 group, hydroxymethyl group, pheny1 group which can be substituted, m or p denotes 0 or 1 respectively, and n denotes an integer of 0 - 2, provided $R^2$ can not be ch1orine atom or hydrogen atom in case both $R^7$ and $R^8$ are hydrogen atom and m is equal to 0, characterized by oxidizing a compound represented by a general formula [II] after a1ka1i-treatment thereof

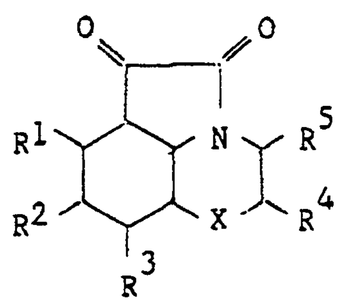

[II]

wherein $R^1$, $R^2$, $R^3$, $R^4$, $R^5$ and x are the same as above-mentioned.

3. A process of preparing cyclic anthranilic acid derivatives, their acid addition salts or alkali salts thereof represented by a general formula [IV]

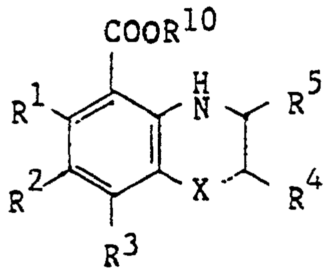

[IV]

wherein $R^1$, $R^2$ and $R^3$, being same or different each other, denote hydrogen atom, halogen atom, lower alkyl group of carbon number 1 - 3, lower alkoxy group of carbon number 1 - 3, amino group, nitro group, hydroxy group, sulfonamide group, trifluoromethyl group, cyano group, carboxyl group, carbamoyl group, acetyl group, benzoylmethyl group which can be substituted, methylthio group, phenylethynyl group which can be substituted, ethynyl group which can be substituted, lower alkanoylamino group of carbon number 1 - 3, benzoylamino group which can be substituted, lower alkylsulfonylamino group of carbon number 1 - 3 or phenylsulfonylamino group which can be substituted, $R^4$ or $R^5$, being same or different each other, denotes hydrogen atom, lower alkyl group of carbon number 1 - 3, cyano group, carboxyl group, hydroxymethyl group, phenyl group which can be substituted or benzyl group which can be substituted, x denotes single bond or denotes $-CHR^7-CHR^8-(CHR^9)m-$, $-O-CHR^8-(CHR^9)m-$, $-S(O)m-CHR^8-(CHR^9)m-$ or $-CO(CHR^8)p-(CHR^9)m-$, $R^{10}$ denotes lower alkyl group of carbon number 1 - 3 or benzyl group, herein $R^7$, $R^8$ or $R^9$, being same or different each other, denotes hydrogen atom, lower alkyl group of carbon number 1 - 3, cyano group, carboxyl group, hydroxymethyl group or phenyl group which can be substituted, m or p denotes 0 or 1 respectively, and n denotes an integer of 0 - 2, provided $R^2$ can not be chlorine atom or hydrogen atom in case both $R^7$ and $R^8$ are hydrogen atom and m is equal to 0, characterized by allowing a coupound represented by a general formula [III]

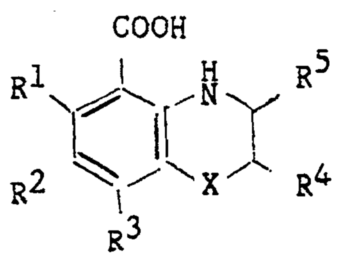

[III]

wherein $R^1$, $R^2$, $R^3$, $R^4$, $R^5$ and x are the same as above-mentioned, to react with lower alkyl halide or benzyl halide.

4. A process of preparing cyclic anthranilic acid derivatives their acid addition salts or alkali salts thereof represented by a general formula [III]

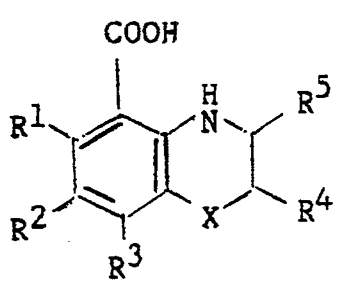

[III]

wherein $R^1$, $R^2$ and $R^3$, being same or different each other, denote hydrogen atom, halogen atom,

lower alkyl group of carbon number l - 3, lower alkoxy group of carbon number 1 - 3, amino group, nitro group, hydroxy group, sulfonamide group, trifuoromethyl group, cyano group, carboxyl group, carbamoyl group, acetyl group, benzoylmethyl group which can be substituted, methylthio group, phenylethynyl group which can be substituted, ethynyl group which can be substituted, lower alkanoylamino group of carbon number 1 - 3, benzoylamino group which can be substituted, lower alkylsulfonylamino group of carbon number 1 - 3 or phenylsulfonylamino group which can be substituted, $R^4$ or $R^5$, being same or different each other, denotes hydrogen atom, lower alkyl group of carbon number 1 - 3, cyano group, carboxyl group, hydroxymethyl group, phenyl group which can be substituted or benzyl group which can be substituted, x denotes single bond or denotes - $CHR^7$ - $CHR^8$ - $(CHR^9)m$ -, - O - $CHR^8$ - $(CHR^9)m$ -, - S(O)n - $CHR^8$ - $(CHR^9)m$ - or - CO $(CHR^8)p$ - $(CHR^9)m$ -, herein $R^7$, $R^8$ or $R^9$, being same or different each other, denotes hydrogen atom, lower alkyl group of carbon number l - 3, cyano group, carboxyl group, hydroxymethyl group or phenyl group which can be substituted, m or p denotes 0 or 1 respectively, and n denotes an integer of 0 - 2, provided $R^2$ can not be chlorine atom or hydrogen atom in case both $R^7$ and $R^8$ are hydrogen atom and m is equal to 0, characterized by subjecting a compound represented by a general formula [IV]

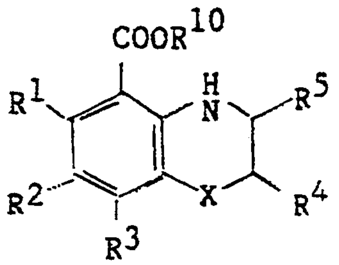

[IV]

wherein $R^1$, $R^2$, $R^3$, $R^4$ and $R^5$ are the same as above-mentioned, $R^{10}$ denotes lower alkyl group of carbon number 1 - 3 or benzyl group, to hydrolysis or catalytic reduction.

5. A process of preparing cyclic anthranilic acid derivatives, their acid addition salts or alkali salts thereof represented by a general formula [VI]

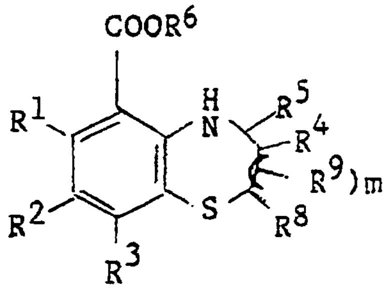

[VI]

wherein $R^1$, $R^2$ and $R^3$, being same or different each other, denote hydrogen atom, halogen atom, lower alkyl group of carbon number 1 - 3, lower alkoxy group of carbon number 1 - 3, amino group, nitro group, hydroxyl group, sulfonamide group, trifluoromethyl group, cyano group, carboxyl group, carbamoyl group, acetyl group, benzoylmethyl group which can be substituted, methylthio group, phenylethynyl group which can be substituted, ethynyl group which can be substituted, lower alkanoylamino group of carbon number 1 - 3, benzoylamino group which can be substituted, lower alkylsulfonylamino group of carbon number 1 - 3 or phenylsulfonylamino group which can be substituted, $R^4$, $R^5$, $R^8$ or $R^9$, being same or different each other, denotes hydrogen atom, lower alkyl group of carbon number l - 3, cyano group, carboxyl group, hydroxymethyl group, phenyl group which can be substituted or benzyl group which can be substituted, $R^6$ denotes hydrogen atom, lower alkyl group of carbon number l - 3 or benzyl group, m denotes 0 or l, characterized by reducing a compound represented by a general formula [V]

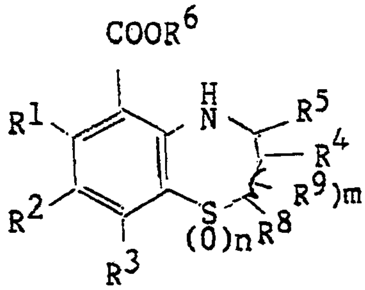

[V]

wherein $R^1$, $R^2$, $R^3$, $R^4$, $R^5$, $R^6$, $R^8$, $R^9$ and m are the same as above, n denotes I or 2.

6. A process of preparing cyclic anthranilic acid derivatives, their acid addition salts or alkali salts thereof represented by a general formula [V]

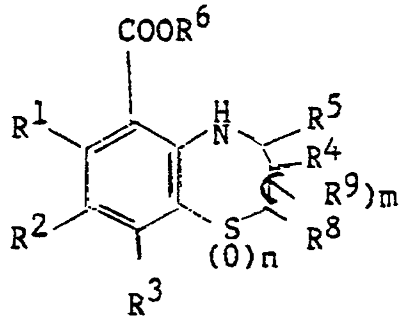

[V]

wherein $R^1$, $R^2$ and $R^3$, being same or different each other, denote hydrogen atom, halogen atom, lower alkyl group of carbon number 1 - 3, lower alkoxy group of carbon number 1 - 3, amino group, nitro group, hydroxyl group, sulfonamide group, trifluoromethyl group, cyano group, carboxyl group, carbomoyl group, acetyl group, benzoylmethyl group which can be substituted, methylthio group, phenylethynyl group which can be substituted, ethynyl group which can be substituted, lower alkanoylamino group of carbon number 1 - 3, benzoylamino group which can be substituted, lower alkylsulfonylamino group of carbon number 1 - 3 or phenylsulfonylamino group which can be substituted, $R^4$, $R^5$, $R^8$ or $R^9$, being same or different each other, denotes hydrogen atom, lower alkyl group of carbon number 1 - 3, cyano group, carboxyl group, hydroxymethyl group, phenyl group which can be substituted or benzyl group which can be substituted, $R^6$ denotes hydrogen atom, lower alkyl group of carbon number 1 - 3 or benzyl group, m denotes 0 or I and n denotes 1 or 2, characterized by oxidizing a compound represented by a general formula [VI]

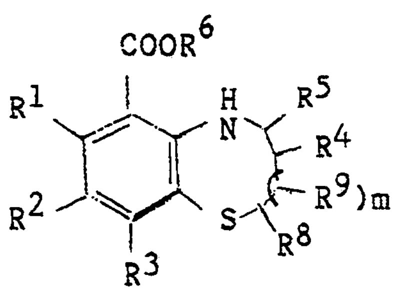

[VI]

wherein $R^1$, $R^2$, $R^3$, $R^4$, $R^5$, $R^6$, $R^8$, $R^9$ and m are the same as above.

7. A process of preparing cyclic anthranilic acid derivatives, their acid addition salts or alkali salts thereof represented by a general formula [VIII]

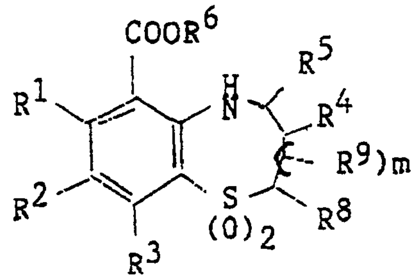

[VIII]

wherein $R^1$, $R^2$ and $R^3$, being same or different each other, denote hydrogen atom, halogen atom, lower alkyl group of carbon number I - 3, lower alkoxy group of carbon number 1 - 3, amino group, nitro group, hydroxyl group, sulfonamide group, trifluoromethyl group, cyano group, carboxyl group, carbamoyl group, acetyl group, benzoylmethyl group which can be substituted, methylthio group, phenylethynyl group which can be substituted, ethynyl group which can be substituted, lower alkanoylamino group of carbon number 1 - 3, benzoylamino group which can be substituted, lower alkylsulfonylamino group of carbon number 1 - 3 or phenylsulfonylamino group which can be substituted, $R^4$, $R^5$, $R^8$ or $R^9$, being same or different each other, denotes hydrogen atom, lower alkyl group of carbon number 1 - 3, cyano group, carboxyl group, hydroxymethyl group, phenyl group which can be substituted or benzyl group which can be substituted, $R^6$ denotes hydrogen atom, lower alkyl group of carbon number 1 - 3 or benzyl group, and m denotes 0 or 1, characterized by oxidizing a compound represented by a general formula [VII]

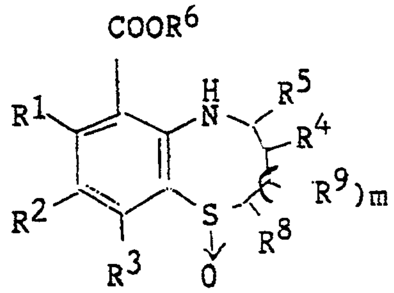

[VII]

wherein $R^1$, $R^2$, $R^3$, $R^4$, $R^5$, $R^6$, $R^8$, $R^9$ and m are the same as above.

8. A process of preparing cyclic anthranilic acid derivatives, their acid addition salts or alkali salts thereof represented by a general formula [X]

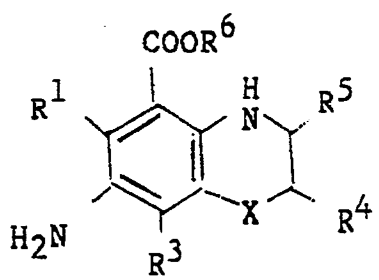

[X]

wherein $R^1$ and $R^3$, being same or different each other, denote hydrogen atom, halogen atom, lower alkyl group of carbon number I - 3, lower alkoxy group of carbon number 1 - 3, amino group, nitro group, hydroxyl group, sulfonamide group, trifluoromethyl group, cyano group, carboxyl group, carbamoyl group, acetyl group, benzoylmethyl group which can be substituted, methylthio group, phenylethynyl group which can be substituted, ethynyl group which can be substituted, lower alkanoylamino group of carbon number I - 3, benzoylamino group which can be substituted, lower alkylsulfonylamino group of carbon number I - 3 or phenylsulfonylamino group which can be substituted, $R^4$ or $R^5$, being same or different each other, denotes hydrogen atom, lower alkyl group of

carbon number I - 3, cyano group, carboxyl group, hydroxymethy1 group, pheny1 group which can be substituted or benzyl group which can be substituted, $R^6$ denotes hydrogen atom, lower a1kyl group of carbon number 1 - 3 or benzyl group, x denotes sing1e bond or denotes - $CHR^7$ - $CHR^8$ - $(CHR^9)m$ - , - O - $CHR^8$ - $(CHR^9)m$ -, - S(O)n - $CHR^8$ - $(CHR^9)m$ -

or -CO $(CHR^8)p$ - $(CHR^9)m$ -, herein $R^7$, $R^8$ or $R^9$, being same or different each other, denotes hydrogen atom, lower alkyl group of carbon number 1 - 3, cyano group, carboxyl group, hydroxymethyl group or phenyl group which can be substituted, m or p denotes 0 or 1 respectively, and n denotes an integer of 0 - 2, characterized by reducing a compound represented by a genera1 formu1a [IX]

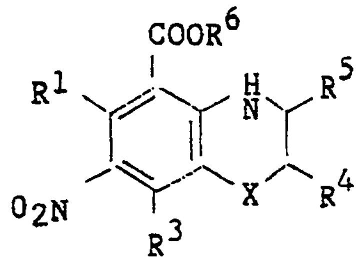

[IX]

wherein $R^1$, $R^3$, $R^4$, $R^5$, $R^6$ and x are the same as above-mentioned.

9. A process of preparing cyclic anthranilic acid derivatives, their acid addition sa1ts or a1ka1i salts thereof represented by a general formu1a [XII]

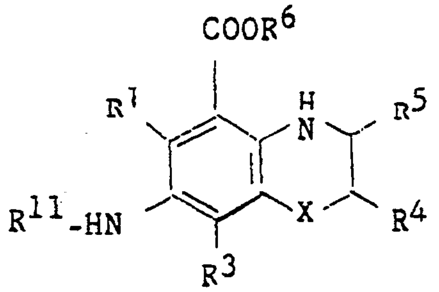

[XII]

wherein $R^1$ and $R^3$, being same or different each other, denote hydrogen atom, ha1ogen atom, 1ower alkyl group of carbon number 1 - 3, lower a1koxy group of carbon number 1 - 3, amino group, nitro group, hydroxy1 group, sulfonamide group, trif1uoromethyl group, cyano group, carboxyl group, carbamoyl group, acetyl group, benzoy1methy1 group which can be substituted, methylthio group, pheny1ethyny1 group which can be substituted, ethyny1 group which can be substituted, lower a1kanoy1amino group of carbon number 1 - 3, benzoylamino group which can be substituted, 1ower a1ky1su1fony1amino group of carbon number I - 3 or phenylsulfonylamino group which can be substituted, $R^4$ or $R^5$, being some or different each other, denotes hydrogen atom, lower alkyl group of carbon number I - 3, cyano group, carboxyl group, hydroxymethyl group, phenyl group which can be substituted or benzyl group which can be substituted, $R^6$denotes hydrogen atom, lower alkyl group of carbon number 1 - 3 or benzyl group, x denotes sing1e bond or denotes - $CHR^7$ - $CHR^8$ - $(CHR^9)m$ -, - O - $CHR^8$ - $(CHR^9)m$ -, - S(O)n - $CHR^8$ - $(CHR^9)m$ -

or - CO $(CHR^8)p$ - $(CHR^9)m$ -, herein $R^7$, $R^8$ or $R^9$, being same or different each other, denotes hydrogen atom, lower alkyl group of carbon number I - 3, cyano group, carboxyl group, hydroxymethy1 group or phenyl group which can be substituted, m or p denotes 0 or 1 respectively, n denotes an integer of 0 - 2, and $R^{11}$ denotes lower a1kanoy1 group of carbon number I - 3, benzoyl group which can be substituted, lower alkylsulfonyl group of carbon number I - 3 or phenylsulfonyl group which can be substituted, characterized by acting a compound represented by a general formula [XI]

$R^{11}$ - Y (XI]

wherein $R^{11}$ is the same as the above, and Y denotes halogen atom, on a compound represented by a general formula [X]

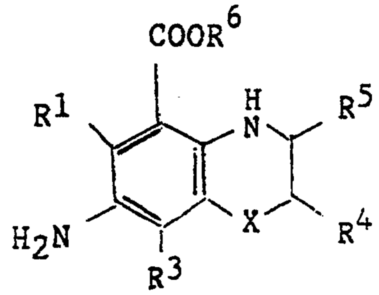

[X]

wherein $R^1$, $R^3$, $R^4$, $R^5$, $R^6$ and x are the same as above-mentioned.

**10.** A process of preparing cyclic anthranilic acid derivatives, their acid addition salts or alkali salts thereof represented by a general formula [XIV]

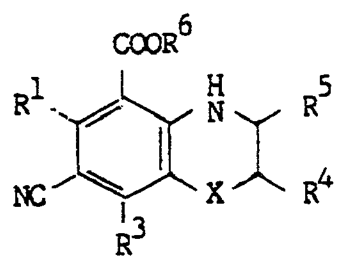

wherein $R^1$ and $R^3$, being same or different each other, denote hydrogen atom, halogen atom, lower alkyl group of carbon number 1 - 3, lower alkoxy group of carbon number 1 - 3, amino group, nitro group, hydroxyl group, sulfonamide group, trifluoromethyl group, cyano group, carboxyl group, carbamoyl group, acetyl group, benzoylmethyl group which can be substituted, methylthio group, phenylethynyl group which can be substituted, ethynyl group which can be substituted, lower alkanoylamino group of carbon number 1 - 3, benzoylamino group which can be substituted, lower alkylsulfonylamino group of carbon number 1 - 3 or phenylsulfonylamino group which can be substituted, $R^4$ or $R^5$, being same or different each other, denotes hydrogen atom, lower alkyl group of carbon number 1 - 3, cyano group, carboxyl group, hydroxymethyl group, phenyl group which can be substituted or benzyl group which can be substituted, $R^6$ denotes hydrogen atom, lower alkyl group of carbon number 1 - 3 or benzyl group, X denotes single bond or denotes $CHR^7$ $CHR^8$ $(CHR^9)m$ , -O $CHR^8$ $(CHR^9)m$ , -S(O)n-$CHR^8$ - $(CHR^9)m$ - or -CO $(CHR^8)p$ - $(CHR^9)m$ -, herein $R^7$, $R^8$ or $R^9$, being same or different each other, denotes hydrogen atom, lower alkyl group of carbon number 1 - 3, cyano group, carboxyl group, hydroxymethyl group or phenyl group which can be substituted, m or p denotes 0 or I respectively, and n denotes on integer of 0 - 2, characterized by converting a compound represented by a general formula [XIII)

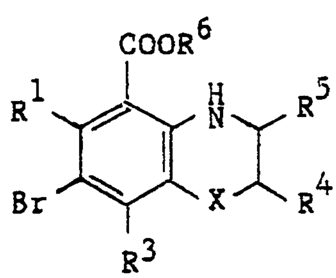

[XIII]

wherein $R^1$, $R^3$, $R^4$, $R^5$ and $R^6$ are the same as above-mentioned, to a nitril derivative thereof.

**11.** A process of preparing cyclic anthranilic acid derivatives, their acid addition salts or alkali salts thereof represented by a general formula [XV]

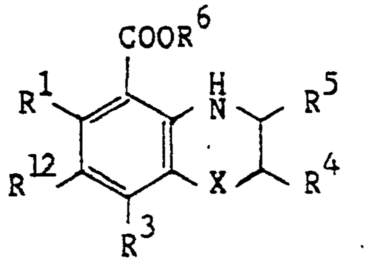

[XV]

wherein $R^1$ and $R^3$, being same or different each other, denote hydrogen atom, halogen atom, lower alkyl group of carbon number 1 - 3, lower alkoxy group of carbon number I - 3, amino group, nitro group, hydroxyl group, sulfonamide group, trifluoromethyl group, cyano group, carboxyl group, carbamoyl group, acetyl group, benzoylmethyl group which can be substituted, methylthio group, phenylethynyl group which can be substituted, ethynyl group which can be substituted, lower alkanoylamino group of carbon number I - 3, benzoylamino group which can be substituted, lower alkylsulfonylamino group of carbon number 1 - 3 or phenylsulfonylamino group which can be substituted, $R^4$ or $R^5$, being same or different each other, denotes hydrogen atom, lower alkyl group of carbon number 1 - 3, cyano group, carboxyl group, hydroxymethyl group, phenyl group which can be substituted or benzyl group which can be substituted, $R^6$ denotes hydrogen atom, lower alkyl group of carbon number I - 3 or benzyl group, X denotes single bond or denotes $-CHR^7$ $-CHR^8$ - $(CHR^9)m$ -, $-O-CHR^8$ - $(CHR^9)m$ -, -S(O)n- $CHR^8$ - $(CHR^9)m$ - or -CO $(CHR^8)p$ - $(CHR^9)m$ -, herein $R^7$, $R^8$ or $R^9$, being same or different each other, denotes hydrogen atom. lower alkyl group of carbon number I - 3. cyano group, carboxyl group, hydroxymethyl group or phenyl group which can be substituted, m or p denotes 0 or I respectively, n denotes an integer of 0 - 2, and $R^{12}$ denotes carbamoyl group or carboxyl group, characterized by hydrolizing a compound represented by a general formula [XIV]

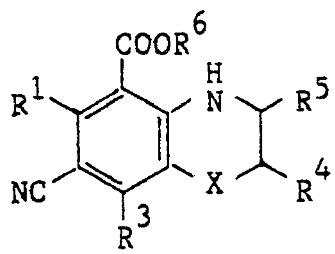

[XIV]

wherein $R^1$, $R^3$, $R^4$, $R^5$ and $R^6$ are the same as above-mentioned.

12. A process of preparing cyclic anthranilic acid derivatives, their acid addition salts or alkali salts thereof represented by a general formula [XVII]

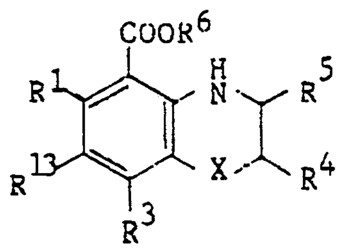

[XVII]

wherein $R^1$ and $R^3$, being same or different each other, denote hydrogen atom, halogen atom, lower alkyl group of carbon number 1 - 3, lower alkoxy group of carbon number 1 - 3, amino group, nitro group, hydroxyl group, sulfonamide group, trifluoromethyl group, cyano group, carboxyl group, carbamoyl group, acetyl group, benzoylmethyl group which can be substituted, methylthio group, phenylethynyl group which can be substituted, ethynyl group which can be substituted, lower alkanoylamino group of carbon number I - 3, benzoylamino group which can be substituted, lower

alkylsulfonylamino group of carbon number l - 3 or phenylsulfonylamino group which can be substituted, $R^4$ or $R^5$, being same or different each other, denotes hydrogen atom, lower alkyl group of carbon number l - 3, cyano group, carboxyl group, hydroxymethyl group , phenyl group which can be substituted or benzyl group which can be substituted, $R^6$ denotes hydrogen atom, lower alkyl group of carbon number 1 - 3 or benzyl group, X denotes single bond or denotes $-CHR^7$ $-CHR^8$ - $(CHR^9)m$ -, $-O-CHR^8$ - $(CHR^9)m$ -, -S(O)n- $CHR^8$ - $(CHR^9)m$ - or -CO $(CHR^8)p$ - $(CHR^9)m$ -, herein $R^7$, $R^8$ or $R^9$, being same or different each other, denotes hydrogen atom, lower alkyl group of carbon number l - 3, cyano group, carboxyl group, hydroxymethyl group or phenyl group which can be substituted, m or p denotes 0 or 1 respectively, n denotes an integer of 0 - 2, $R^{13}$ denotes phenylethynyl group which can be substituted or ethynyl group which can be substituted, characterized by acting phenylacetylene which can be substituted or acetylene which can be substituted on a compound represented by a general formula [XVI]

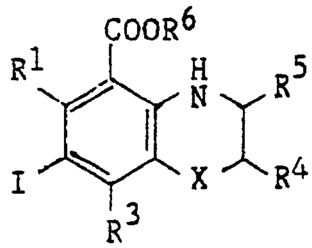

[XVI]

wherein $R^1$ , $R^3$, $R^4$, $R^5$, $R^6$ and x are the same as above-mentioned.

**13.** A process of preparing cyclic anthranilic acid derivatives, their acid addition salts or alkali salts thereof represented by a general formula [XVIII]

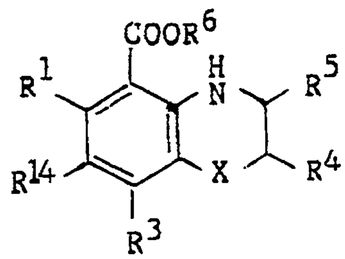

[XVIII]

wherein $R^1$ and $R^3$, being same or different each other, denote hydrogen atom, halogen atom, lower alkyl group of carbon number 1 - 3, lower alkoxy group of carbon number 1 - 3, amino group, nitro group, hydroxyl group, sulfonamide group, trifluoromethyl group, cyano group, carboxyl group, carbamoyl group, acetyl group, benzoylmethyl group which can be substituted, methylthio group, phenylethynyl group which can be substituted, ethynyl group which can be substituted, lower alkanoylamino group of carbon number l - 3, benzoylamino group which can be substituted, lower alkylsulfonylamino group of carbon number l - 3 or phenylsulfonylamino group which can be substituted, $R^4$ or $R^5$, being same or different each other, denotes hydrogen atom, lower alkyl group of carbon number 1 - 3, cyano group, carboxyl group, hydroxymethyl group, phenyl group which can be substituted or benzyl group which can be substituted , $R^6$ denotes hydrogen atom, lower alkyl group of carbon number l - 3 or benzyl group, X denotes single bond or denotes $-CHR^7$ $-CHR^8$ - $(CHR^9)m$ -, $-O-CHR^8$ - $(CHR^9)m$ -, $-S(O)n-CHR^8$ - $(CHR^9)m$ -

or -CO $(CHR^8)p$ - $(CHR^9)m$ -, herein $R^7$, $R^8$ or $R^9$, being same or different each other, denotes hydrogen atom, lower alkyl group of carbon number l - 3, cyano group, carboxyl group, hydroxymethyl group or phenyl group which can be substituted, m or p denotes 0 or 1 respectively, n denotes an integer of 0 - 2, and $R^{14}$ denotes benzoylmethyl group which can be substituted or acetyl group, characterized by hydrating a compound represented by a general formula [XVII]

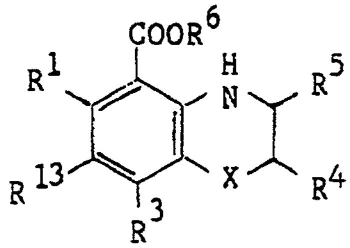

[XVII]

wherein $R^1$, $R^3$, $R^4$, $R^5$, $R^6$, and x are the same as above-mentioned, and $R^{13}$ denotes pheny1ethynyl group which can be substituted or ethynyl group which can be subsituted.

**14.** Antirheumatic drug, antoimmune disease curing drug and metabo1ic bone disease curing drug, comprising at least one compound of cyc1ic anthrani1ic acid derivatives, their acid addition sa1ts or their alka1i salts represented by a general formula [I] as an active ingredient.

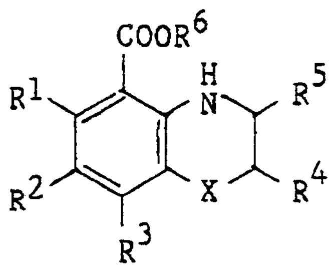

[I]

wherein $R^1$, $R^2$ and $R^3$, being same or different each other, denote hydrogen atom, halogen atom, 1ower a1kyl group of carbon number l - 3, lower alkoxy group of carbon number 1 - 3, amino group, nitro group, hydroxyl group, sulfonamide group, trifluoromethyl group, cyano group, carboxy1 group, carbamoy1 group, acety1 group, benzoylmethyl group which can be substituted, methylthio group, phenylethynyl group which can be substituted, ethyny1 group which can be substituted, 1ower a1kanoy1amino group of carbon number 1 - 3, benzoy1amino group which can be substituted, 1ower a1ky1su1fony1amino group of carbon number 1 - 3 or phenylsulfonylamino group which can be substituted, $R^4$ or $R^5$, being same or different each other, denotes hydrogen atom, lower alkyl group of carbon number l - 3, cyano group, carboxyl group, hydroxymethyl group, phenyl group which can be substituted or benzyl group which can be substituted, $R^6$ denotes hydrogen atom, lower alky1 group of carbon number l - 3 or benzyl group, X denotes sing1e bond or denotes -$CHR^7$ -$CHR^8$ - $(CHR^9)m$ -, -O-$CHR^8$ - $(CHR^9)m$ -, -S(O)n -$CHR^8$-$(CHR^9)m$ - or CO $(CHR^8)p$ - $(CHR^9)m$ -, herein $R^7$, $R^8$ or $R^9$, being same or different each other, denotes hydrogen atom, lower a1ky1 group of carbon number 1 - 3, cyano group, carboxy1 group, hydroxymethy1 group or phenyl group which can be substituted, m or p denotes 0 or 1 respective1y, and n denotes an integer of 0 - 2, provided $R^2$ can not be chlorine atom or hydrogen atom in case both $R^7$ and $R^8$ are hydrogen atom and m is equal to 0.

# INTERNATIONAL SEARCH REPORT

International Application No PCT/JP90/00823

**I. CLASSIFICATION OF SUBJECT MATTER** (if several classification symbols apply, indicate all) [6]

According to International Patent Classification (IPC) or to both National Classification and IPC Int. $Cl^5$ C07D209/08, C07D209/12, C07D209/42, C07D223/16, C07D225/06, C07D267/14, C07D267/22, C07D281/10, C07D281/18, A61K31/395, A61K31/40, A61K31/55

**II. FIELDS SEARCHED**

Minimum Documentation Searched [7]

| Classification System | Classification Symbols |
|---|---|
| IPC | C07D209/08, C07D209/12, C07D209/42, C07D223/16, C07D225/06, C07D267/14, C07D267/22, C07D281/10, C07D281/18, A61K31/395, A61K31/40, A61K31/55 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | JP, A, 46-4827 (A.H. Robins Co., Inc.), 19 November 1971 (19. 11. 71), DE, A, 2117116 & FR, A, 2092043 | 1 - 13 |
| Y | JP, A, 46-4827 (A.H. Robins Co., Inc.), 19 November 1971 (19. 11. 71), DE, A, 2117116 & FR, A, 2092043 | 1 - 14 |
| X | JP, A, 62-238288 (Nippon Tokushu Noyaku K.K.), 19 October 1987 (19. 10. 87), EP, A, 239927 & US, A, 4729998 | 1 - 13 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| September 3, 1990 (03. 09. 90) | September 17, 1990 (17. 09. 90) |
| **International Searching Authority** | **Signature of Authorized Officer** |
| Japanese Patent Office | |